# EUROPEAN PATENT APPLICATION

(11) **EP 2 243 833 A1**
(43) Date of publication of application: **27.10.2010**
(21) Application number: 09158496.1
(22) Date of filing: 22.04.2009
(51) Int. Cl.: C12N 15/11, A61K 31/7088, A61P 35/02

(54) **Method for the treatment of acute myeloid leukemia**

(71) Applicant: Erasmus University Medical Center Rotterdam, 3015 GD Rotterdam (NL)
(72) Inventor: Löwenberg, Bob, 3062 JP Rotterdam (NL); Lavrencic, Mojca, 3008 AE Rotterdam (NL); Valk, Peter Jacobus Maria, 3068 TD Rotterdam (NL); Sun, Su Ming, 3012 HG Delft (NL)
(74) Representative: Habets, Winand

(57) **Abstract**

The invention is in the field of molecular medicine and provides methods for the treatment of acute myeloid leukemia. These methods are based on the observation that microRNA 9/9* are involved in the pathogenesis of the disease in that the overexpression of microRNA-9/9* block myeloid differentiation in vitro. More in particular, microRNA-9/9* were found to play a role in leukemic transformation in acute myeloid leukemia.

## Description

### Field of the invention

The invention is in the field of molecular medicine and provides methods for the treatment of acute myeloid leukemia. These methods are based on the observation that microRNA-9 and/or microRNA-9* (microRNA-9/9*) are involved in the pathogenesis of the disease in that the overexpression of microRNA-9/9* block myeloid differentiation in vitro. More in particular, microRNA-9/9* were found to play a role in leukemic transformation in acute myeloid leukemia.

### Background of the invention

Acute myeloid leukemia (AML) is a remarkably heterogeneous malignant disease, characterized by uncontrolled clonal outgrowth of hematopoietic progenitor cells, blocked at different stages of differentiation. The variable clinical biology and prognosis is mainly determined by somatic cytogenetic abnormalities e.g., t(15;17), t(8;21), inv(16), 11q23, 3q26 abnormalities and monosomal karyotype (1-3) and a variety of gene mutations. Prominent examples are mutations in nucleophosmin-1 (NPM1), fms-like tyrosine kinase receptor (FLT3-interenal tandem duplications (ITD)) and CCAAT binding transcription factor CEBPA (4-7). These acquired genetic aberrations may coexist and are thought to cooperate synergistically in the development of leukemia.

The pathogenesis of AML is a multi-step process affecting cell differentiation, proliferation and apoptosis that ultimately lead to malignant transformation of hematopoietic progenitors. The pathogenetic notion is that at least two, probably in most cases multiple, hits are required for leukemic transformation. One category of genetic abnormalities (class 1) results in constitutive activation of proliferative signaling and involves signaling molecules such as RAS, FLT3 and c-KIT. A second type of lesions (class 2) such as formation of fusion genes, as a result of the chromosomal lesions t(8;21) or inv(16) involving transcription factors result in a block of myeloid differentiation. Various studies in the mice models established the cooperation of both types of genetic deregulation in AML development.

There is a wealth of information on aberrantly expressed genes in AML. Usually, diagnosis of AML and its clinical subgroups is based on the expression of gene profiles collectively referred to as classifiers.

It has recently been demonstrated that microRNA expression profiling in AML also reveals highly distinctive microRNA expression profiles (8).

MicroRNAs are a class of small non-coding RNAs that posttranscriptionally regulate gene expression (9). Theyregulate gene expression by forming base pairs with sequences in the 3' untranslated region (3'UTR) of mRNAs thereby repressing translation or inducing degradation of their target mRNA transcripts. To date, more than 400 microRNAs have been described in humans; however, the precise function of these regulatory, non-coding RNAs remains largely obscure.

Differentially expressed microRNAs appeared to characterize particular AML genotypes. Several established oncogenic and tumor suppressor microRNAs, such as microRNA-155, microRNA-21 and let-7 appeared to be associated with specific genetic subtypes of AML(8).

Lu et al., (10)showed that patterns of microRNA gene activity can distinguish between several types of human cancers. They measured the activity of 217 genes encoding microRNA in a new, bead-based flow cytometric microRNA expression profiling method. microRNA signatures may therefore enable classification of cancer. This finding may allow determining the original tissue type which spawned a cancer and targeting a treatment course based on the original tissue type. Lu et al., observed a general downregulation of microRNAs in tumours compared with normal tissues.

Furthermore, they were able to successfully classify poorly differentiated tumours using microRNA expression profiles, whereas messenger RNA profiles were highly inaccurate when applied to the same samples. These findings highlight the potential of microRNA profiling in cancer diagnosis.

Recent studies have shown an association between microRNA expression signatures and (cyto)genetics subsets of AML (8, 11, 12). Furthermore, it was demonstrated that microRNA expression signature containing microRNA-233, - 128a -128b and let7b can accurately distinguish AML from acute lymphoblastic leukemia ALL (13).

Increased expression of microRNA-191 and -199a was associated with poor survival in one AML study (12).

A microRNA signature of cytogenetically normal AML was identified that is predictive for adverse prognosis (14).

Another study showed that the let-7/miR-98 cluster is commonly down-regulated whereas microRNA-21, microRNA-155, microRNA-181 b, microRNA-221 and microRNA-222 are frequently up-regulated in both solid and hematological tumours (15).

Chen and the colleagues (16) were the first that cloned microRNAs from normal mouse bone marrow and found microRNA-223, -142 and -181 preferentially expressed in hematopoietic tissue. MicroRNA-233 was found to be expressed at low levels in CD34+ hematopoietic progenitors (HPCs) and this expression increased during myeloid differentiation towards granulocytes.

Notwithstanding the fact that theseand other studies provide important information for the diagnosis of AML, they do not provide insights for novel therapies for AML, since it has not been established whether the overexpression or underexpression of a particular gene or microRNA is an epiphenomenon or contributes to the pathogenesis of the disease.

Only a few publications have recently appeared on the relevance of microRNAs in the therapy of cancer. A study of mice altered to produce excess c-myc - a protein implicated in several cancers - shows that miRNA 17-92 polycistron has an effect on the development of cancer. Mice that were engineered to produce a surplus of types of microRNA found in lymphoma cells developed the disease within 50 days and died two weeks later. In contrast, mice without the surplus microRNA lived over 100 days(17). These studies indicate that non-coding RNAs, specifically microRNAs, can modulate tumour formation, and implicate the microRNA-17-92 cluster as a potential human oncogene.

MicroRNA-125b overexpression, that is directly linked to t(2;11) appeared to abrogate myeloid cell differentiation in vitro implying a function of this microRNA in leukemogenesis (18).

Overexpression of microRNA-155 in mouse bone marrow expanded the granulocyte/monocyte population with pathological features characteristic of myeloid neoplasia (19).

In AML, upregulation of microRNA-155 is associated with mutations in the gene of the kinase receptor Fms-like tyrosine kinase-3 (Flt3), so called FLT3-ITD.

In a recent study increased expression of microRNA-196b by MLL fusion proteins has been suggested to contribute to development of AML via increased cell proliferation capacity and survival as well as partial block in differentiation in vitro (20).

Another study found that two types of microRNA (miRNA-17-5p and miRNA-20a) inhibit the E2F1 protein, which regulates cell proliferation.

Johnnidis et al (21), using a loss-of-function allele in mice,demonstrated that the microRNA-223 negatively regulates progenitor proliferation and granulocyte differentiation by targeting Mef2c, a transcription factor that promotes myeloid progenitor proliferation.

Using an acute pro-myelocytic leukemia cell-line model, an autoregulation model of microRNA-223, CEBPA and NF1A has been proposed (22). Furthermore, PU.1 was found to activate microRNA-223 transcription in mice (23).

Significantly differentially expressed microRNAs during monocyte/macrophage differentiation of the cell line HL60 were identified (24). Besides results obtained from the cell lines models (25, 26) very limited data is available on microRNA expression profiles during normal myeloid differentiation using primary human cells (27).

Functional studies have demonstrated that let-7/miR-98 negatively regulate RAS(28) and v-myc myelocytomatosis viral oncogene homologue (MYC)(29)whereasmicroRNA-21 negatively regulate programmed cell death 4 (PDCD4)(30)and phosphatase and tensin homologue (PTEN)(31).

Despite of these findings, there remains a need for additional and more specific targets for the treatment of cancers, in particular for the treatment of acute myeloid leukemia (AML).

### Summary of the invention

We identified candidate microRNAs with aberrant expression in AML as compared with normal hematopoietic progenitors. microRNA-9/9* appears strongly overexpressed in a great number of AML samples and is actively involved in the transformation process. microRNA-9/9* therefore provides an attractive target for the therapy of acute myeloid leukemia.

Our experiments show that microRNA-9/9* induces a block in granulocytic differentiation in the murine growth factor dependent myeloid cell line 32D in vitro. This let us to conclude that acute myeloid leukemia may effectively be treated by interfering with the binding between microRNA-9/9* and their targets.

The invention therefore relates to a method for the treatment of AML wherein a therapeutic composition is administered to a patient in need thereof comprising as an active ingredient a compound capable of interacting with the binding between microRNA-9/9* and its target.

### Detailed description of the invention

We found that microRNA-9/9* play a role in leukemic transformation in acute myeloid leukemia.

MicroRNAs are single-stranded RNAs of 18-25 nucleotides and are generated from precursor molecules. microRNAs are encoded in the genome and transcribed by RNA polymerase II as primary transcripts called pri-microRNAs which are processed in the nucleus into one or more precursor-microRNAs (pre-microRNAs) by the nuclear RNase III, Drosha, and the double-stranded RNA binding protein, Pasha/DGCR8 (32, 33). In the cytoplasm, another RNase III, known as Dicer, further processes the pre-microRNA into double-stranded 23-nucleotide mature microRNA. This microRNA duplex (34) comprises a strand (microRNA strand) which is incorporated into the RNA-induced silencing complex (RISC) and a complementary strand (microRNA* strand), which is usually degraded.

There are several exceptions wherein the mature microRNA* strand is also coexpressed. This is the case with microRNA-9, since also microRNA-9* is usually expressed and is not degraded.

The functional RISC carrying the mature microRNA-9 and/or microRNA-9* can bind to the 3'UTR of theirtarget gene mRNA to result in either mRNA degradation or protein translation inhibition (35, 36)

Genes encoding microRNA-9 and microRNA-9* are located on human chromosomes 1 (miR-9-1), 5 (miR-9-2), and 15 (miR-9-3). Upon processing of these different microRNA-9 precursors, the complementarymature microRNA-9 (UCUUUGGUUAUCUAGCUGUAUGA) and microRNA-9* (AUAAAGCUAGAUAACCGAAAGU) are derived.

MicroRNA-9 is enriched in brain and appears important in brain development and lineage commitment (37, 38). In zebrafish, microRNA-9 is important for midbrain-hindbrain boundary definition (39). In mouse cortical development, microRNA-9 appears necessary for appropriate differentiation of Cajal-Retzius cells (40). MicroRNA-9 regulates REST and CoREST and is downregulated in Huntington's Disease (41). microRNA-9 is upregulated in a human neuroblastoma cell line by retinoic acid and is downmodulated in primary neuroblastoma tumors (42).

Increased expression of microRNA-9, which is expressed in beta cells, impairs glucose-stimulated insulin release (43). The latter perturbation of the secretory functions was associated with an increase in the level of granuphilin (SYTL4) a Rab3/Rab27 effector playing a negative modulatory role on insulin exocytosis.

MicroRNA-9 has also been shown to be differentially expressed at successive stages in the course of bronchial carcinogenesis (44). Roccaro el al (45) identified a microRNA signature specific of Waldenstrom Macroglobulinemia with decreased expression of microRNA-9. Recently hypermethylation and decreased expression of microRNA-9 in acute lymphoblastic leukemia (ALL) has been reported correlating with the clinical outcome of these patients (46).

We found that microRNA-9 and microRNA9* areboth significantly overexpressed in primary human AML. We performed a supervised analysis using significance analysis of microarray(SAM).

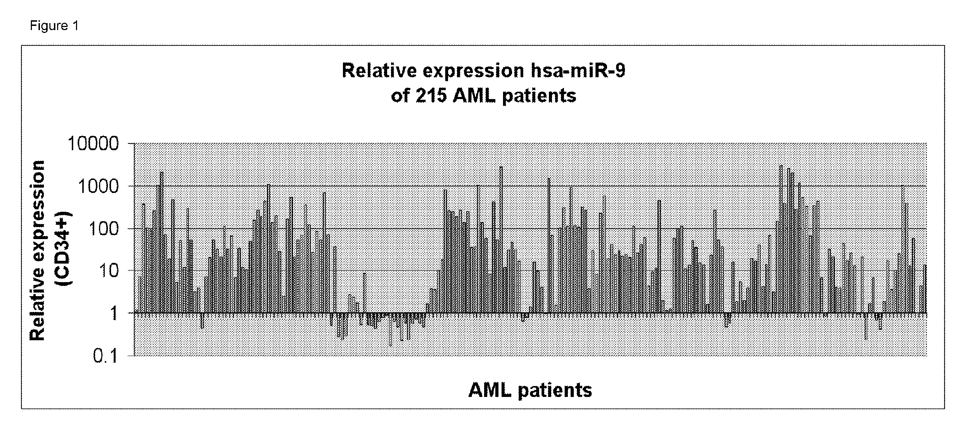

MicroRNA expression of all AML samples was compared to the microRNAs expression patterns of the normal bone marrow CD34+ cells. One of the most discriminating microRNAs weremicroRNA-9 and microRNA-9*, that both appeared to be more than 100 fold upregulated in AML. This is shown in figure 1 for microRNA-9. The results obtained for microRNA-9* were comparable if not identical.

Furthermore, we found that microRNA-9 and microRNA9* werenot expressed during normal myelopoiesis. We studied the role of microRNA-9 and microRNA9* in hematopoietic differentiation in that we evaluated its expression throughout myeloid development in highly purified cell populations from normal bone marrow and during in vitro myeloid differentiation assay of normal human CD34+ cell.

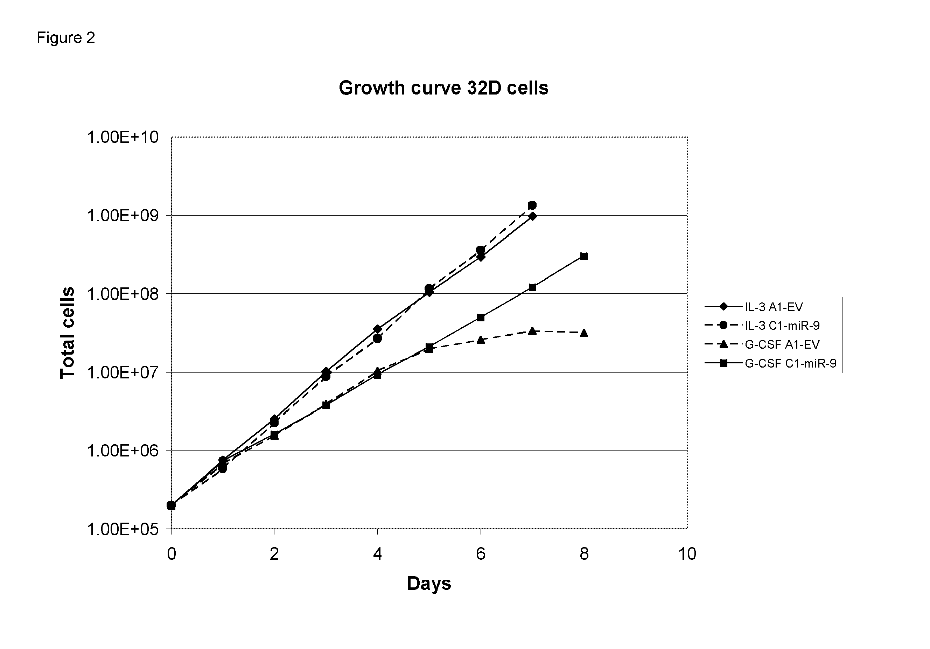

Using both approaches the expression of microRNA-9 and microRNA-9* was at the detection level of the quantitative RT-PCRassay in myeloid progenitors and remained so as granulocytic differentiation proceeds through promyelocytes, metamyelocytes to mature neutrophils. These data show that microRNA-9 and microRNA-9* do not have a function in normal myeloid differentiation. We conclude that the aberrant expression of microRNA-9/9*in AML as detailed above is associated with leukemogenesis rather than that it reflects the differentiation stage of AML. In addition, we found that miRNA-9/9* induces a block in myeloid differentiation of 32D cells (murine myeloid leukemia cell line) and primary murine bone marrow cells in vitro. For that purpose, microRNA-9/9* was retrovirally transducedinto the 32D cell line. This cell line is known to proliferate when interleukin-3 (IL-3) is added to the medium but is able to differentiate towards neutrophils when IL3 is replaced by granulocyte-colony stimulating factor (G-CSF). Using hematocytology (cytospins) cell staining of 32D cells we observed a block in the differentiation under IL-3 conditions and persistent cell proliferation under the G-CSF condition (Figure 2). Furthermore, microRNA-9 and microRNA-9*werealso introduced into primary murine bone marrow cells. microRNA-9/9* overexpression resulted in decreased numbers of mature neutrophils (CD11b+Gr1+ cells) in vitro as defined by flow-cytometry. This corroborates our findings in the 32D cell line.

Cytospin stainings were performed on various timepoints (day 0, 3, 7, 10, 15) during the differentiation assay with G-CSF. 32D cells transduced with control vector were differentiating from blasts on day 0 to completely mature granulocytes on day 7. Interestingly, in 32D cells transduced with microRNA-9/9* we observed block in differentiation, since no mature granulocytes were observed on day 7 and all later time points.

Furthermore, microRNA-9/9* was introduced to the primary murine bone marrow cells. microRNA-9/9* overexpression using retroviral vector resulted in decreased percantage of mature neutrophils (CD11b+Gr1+ cells) in vitro examined by immunophenotyping.

In summary, we found that microRNA-9/9* overexpression results in functional phenotype, namely block in myeloid differentiation. Based on these results we conclude that microRNA-9/9* plays a role in leukemic transformation in AML.

The findings as detailed above make it possible to intervene with the interaction of microRNA-9/9* and their cellular targets, thereby providing a method for the treatment of AML. The invention therefore provides a method for the treatment of AML wherein a therapeutic composition is administered to a patient in need thereof comprising as an active ingredient a compound capable of interacting with the binding between microRNA-9/9* and their targets.

In the alternative, the intervention may be at the level of microRNA-9/9* transcription and/or processing so that the level of mature microRNA-9/9* in the cell is decreased. Hence, the invention also relates to a method for the treatment of AML wherein a therapeutic composition is administered to a patient in need thereof comprising as an active ingredient a compound capable of decreasing the cellular level of microRNA-9/9*.

This may be effected by providing a compound that interferes with the transcription or processing of microRNA-9/9*. The invention therefore preferably relates to a method as described above wherein the compound is capable of interfering with the transcription or processing of precursor microRNA-9.

Compounds, also called antagomirs (Exiqon) and antimirs (Dharmacon), which are capable of interfering with the interaction of microRNA-9 microRNA-9* and their targets are known in the art. Additional compounds may easily be found by using known assays, for instance a Renilla Luciferase assay wherein microRNA-9 is allowed to bind to 3' UTR of candidate wild type and mutated target. Additional compounds useful in the invention may also be found by immunobloting (Western assay) wherein protein levels of candidate microRNA9/9* targets can be examined and compared in cells with or without overexpression of microRNA9/9* in combination with inhibitory compound. The invention therefore also relates to a compound capable of interacting with the binding between microRNA-9/9* and theirtargets for the treatment of AML. Also, the invention relates to a compound capable of decreasing the cellular level of microRNA-9/9* for the treatment of AML.

### Legends to the figures

### Figure 1: microRNA-9 and microRNA9* are significantly overexpressed in primary human AML

SDS 2.3 software (Applied Biosystems) was used to analyze microRNA-9/9* expression data obtained by Real-time RT-PCR. The geometric mean of several snRNAs (small nuclear RNAs) expression was used for internal normalization. The comparative relative quantification method, 2-ddCt was used to calculate the relative expression (fold change) of the microRNA-9 in AMLs compared to normal CD34+ cells. Relative expression (fold change) of microRNA-9 is presented on the vertical axis for all 215 AML samples in the cohort (horizontal axis).

### Figure 2: overexpression of microRNA-9/9* in 32D cell line leads to block in myeloid differentiation.

MicroRNA-9/9* or control vector were transduced into the murine myeloid leukemia cell line (32D) using retroviral vector containing GFP (green florescence protein) reporter. Cells with high GFP expression were sorted and used in a proliferation/differentiation assay. Overexpression of microRNA-9/9* had no effect on proliferating capacity of 32D cells growing in IL-3 supplemented medium. However, when growing them in G-CSF containing medium, microRNA-9/9* transduced cells continue with proliferation while cells transduced with control vector decreased in grow.

### Examples

### Example 1: Testing the activity of new potential microRNA9/9* inhibitory compound using a known microRNA-9 target onecut2 (OC2)

Luciferase assay: Onecut2 (OC2) transcription factor was previously identified as a target of microRNA-9 (43). To generate the 3'-UTR-OC2-luc construct, the 3'-UTR segment of the rat oc2 gene must be amplified by PCR from genomic DNA and inserted in the multiple cloning site of the psiCHECK-1 plasmid (Promega, Madison, WI). The multiple cloning site of this plasmid is located in the 3'-UTR of the Renilla luciferase gene between the stop codon and an artificial polyadenylation site. The sequences of the PCR primers are as follows: sense, 5'-GGATGTCTCGAGTGTTTTCTACAAAG-3', (SEQ ID NO: 1) and antisense, 5'-GAAGCAGCGGCCGTTGAGGCTCCTC-3' (SEQ ID NO: 2). The microRNA-9-sensor construct can be obtained by cloning the microRNA-9 mature sequence in the antisense orientation in the 3'-UTR of the Renilla luciferase gene of psiCHECK-1 (Promega).

Transient transfections of plasmids are than performed using the Effectene transfection kit (Qiagen, Valencia, CA). Experiments involving transient transfections of microRNA-9 and control microRNA can be carried out with Lipofectamine 2000 (Invitrogen) using 100 nM RNA duplexes. Luciferase activities is measured 2 days after transfection with the dual-luciferase reporter assay system (Promega). For 3'-UTR-OC2-luc and microRNA-9-sensor co-transfections with the Firefly luciferase SV40 pGL3 promoter (Promega) or with the Renilla luciferase pRLCMV (pRLrenilla), respectively, can be used for normalization.

Immunoblot Analysis:For Western blot analysis, the cells must be washed once in icecold phosphate-buffered saline, protein extracts are prepared as described before (43), 15 µg of proteins is subjected to SDS-PAGE and transferred on polyvinylidene difluoride membranes. The membranes are blocked for 60 min in a buffer containing 0.1% Tween 20 and 5% milk and were then incubated overnight at 4 °C with a primary antibody raised against rat OC2 (amino acids 36-311) (32). Immunoreactive bands must be visualized using a chemiluminescent substrate (Amersham Biosciences) after incubation of the membrane for 60 min with secondary antibody conjugated to horseradish peroxidase.

### References

1. Slovak ML, Kopecky KJ, Cassileth PA, Harrington DH, Theil KS, Mohamed A, Paietta E, Willman CL, Head DR, Rowe JM, et al. (2000) Blood96, 4075-4083.
2. Grimwade D, Walker H, Harrison G, Oliver F, Chatters S, Harrison CJ, Wheatley K, Burnett AK, & Goldstone AH (2001) Blood98, 1312-1320.
3. Breems DA, Van Putten WL, De Greef GE, Van Zelderen-Bhola SL, Gerssen-Schoorl KB, Mellink CH, Nieuwint A, Jotterand M, Hagemeijer A, Beverloo HB, et al. (2008) J Clin Oncol26, 4791-4797.
4. Dohner K, Schlenk RF, Habdank M, Scholl C, Rucker FG, Corbacioglu A, Bullinger L, Frohling S, & Dohner H (2005) Blood106, 3740-3746.
5. Frohling S, Schlenk RF, Breitruck J, Benner A, Kreitmeier S, Tobis K, Dohner H, & Dohner K (2002) Blood100, 4372-4380.
6. Barjesteh van Waalwijk van Doorn-Khosrovani S, Erpelinck C, Meijer J, van Oosterhoud S, van Putten WL, Valk PJ, Berna Beverloo H, Tenen DG, Lowenberg B, & Delwel R (2003) Hematol J4, 31-40.
7. Falini B, Nicoletti I, Martelli MF, & Mecucci C (2007) Blood109, 874-885.
8. Jongen-Lavrencic M, Sun SM, Dijkstra MK, Valk PJ, & Lowenberg B (2008) Blood111, 5078-5085.
9. Bartel DP (2004) Cell116, 281-297.
10. Lu J, Getz G, Miska EA, Alvarez-Saavedra E, Lamb J, Peck D, Sweet-Cordero A, Ebert BL, Mak RH, Ferrando AA, et al. (2005) Nature435, 834-838.
11. Li Z, Lu J, Sun M, Mi S, Zhang H, Luo RT, Chen P, Wang Y, Yan M, Qian Z, et al. (2008) Proc Natl Acad Sci U S A105, 15535-15540.
12. Garzon R, Volinia S, Liu CG, Fernandez-Cymering C, Palumbo T, Pichiorri F, Fabbri M, Coombes K, Alder H, Nakamura T, et al. (2008) Blood111, 3183-3189.
13. Mi S, Lu J, Sun M, Li Z, Zhang H, Neilly MB, Wang Y, Qian Z, Jin J, Zhang Y, et al. (2007) Proc Natl Acad Sci U S A104, 19971-19976.
14. Marcucci G, Radmacher MD, Maharry K, Mrozek K, Ruppert AS, Paschka P, Vukosavljevic T, Whitman SP, Baldus CD, Langer C, et a/. (2008) N Engl J Med358, 1919-1928.
15. Volinia S, Calin GA, Liu CG, Ambs S, Cimmino A, Petrocca F, Visone R, lorio M, Roldo C, Ferracin M, et al. (2006) Proc Natl Acad Sci U S A103, 2257-2261.
16. Chen CZ, Li L, Lodish HF, & Bartel DP (2004) Science303, 83-86.
17. He L, Thomson JM, Hemann MT, Hernando-Monge E, Mu D, Goodson S, Powers S, Cordon-Cardo C, Lowe SW, Hannon GJ, et al. (2005) Nature435, 828-833.
18. Bousquet M, Quelen C, Rosati R, Mansat-De Mas V, La Starza R, Bastard C, Lippert E, Talmant P, Lafage-Pochitaloff M, Leroux D, et al. (2008) J Exp Med205, 2499-2506.
19. O'Connell RM, Rao DS, Chaudhuri AA, Boldin MP, Taganov KD, Nicoll J, Paquette RL, & Baltimore D (2008) J Exp Med205, 585-594.
20. Popovic R, Riesbeck LE, Velu CS, Chaubey A, Zhang J, Achille NJ, Erfurth FE, Eaton K, Lu J, Grimes HL, et al. (2009) Blood*.*
21. Johnnidis JB, Harris MH, Wheeler RT, Stehling-Sun S, Lam MH, Kirak O, Brummelkamp TR, Fleming MD, & Camargo FD (2008) Nature451, 1125-1129.
22. Fazi F, Rosa A, Fatica A, Gelmetti V, De Marchis ML, Nervi C, & Bozzoni I (2005) Cell123, 819-831.
23. Fukao T, Fukuda Y, Kiga K, Sharif J, Hino K, Enomoto Y, Kawamura A, Nakamura K, Takeuchi T, & Tanabe M (2007) Cell129, 617-631.
24. Fontana L, Pelosi E, Greco P, Racanicchi S, Testa U, Liuzzi F, Croce CM, Brunetti E, Grignani F, & Peschle C (2007) Nat Cell Biol9, 775-787.
25. Shi B, Prisco M, Calin G, Liu CG, Russo G, Giordano A, & Baserga R (2006) J Cell Physiol207, 706-710.
26. Garzon R, Pichiorri F, Palumbo T, Visentini M, Aqeilan R, Cimmino A, Wang H, Sun H, Volinia S, Alder H, et al. (2007) Oncogene26, 4148-4157.
27. Georgantas RW, 3rd, Hildreth R, Morisot S, Alder J, Liu CG, Heimfeld S, Calin GA, Croce CM, & Civin Cl (2007) Proc Natl Acad Sci U S A104, 2750-2755.
28. Johnson SM, Grosshans H, Shingara J, Byrom M, Jarvis R, Cheng A, Labourier E, Reinert KL, Brown D, & Slack FJ (2005) Cell120, 635-647.
29. Sampson VB, Rong NH, Han J, Yang Q, Aris V, Soteropoulos P, Petrelli NJ, Dunn SP, & Krueger LJ (2007) Cancer Res67, 9762-9770.
30. Lu Z, Liu M, Stribinskis V, Klinge CM, Ramos KS, Colburn NH, & Li Y (2008) Oncogene27, 4373-4379.
31. Meng F, Henson R, Wehbe-Janek H, Ghoshal K, Jacob ST, & Patel T (2007) Gastroenterology133, 647-658.
32. Saini HK, Griffiths-Jones S, & Enright AJ (2007) Proc Natl Acad Sci U S A104, 17719-17724.
33. Gregory RI, Yan KP, Amuthan G, Chendrimada T, Doratotaj B, Cooch N, & Shiekhattar R (2004) Nature432, 235-240.
34. Ketting RF, Fischer SE, Bernstein E, Sijen T, Hannon GJ, & Plasterk RH (2001) Genes Dev15, 2654-2659.
35. Okamura K, Ishizuka A, Siomi H, & Siomi MC (2004) Genes Dev18, 1655-1666.
36. Liu J, Carmell MA, Rivas FV, Marsden CG, Thomson JM, Song JJ, Hammond SM, Joshua-Tor L, & Hannon GJ (2004) Science305, 1437-1441.
37. Krichevsky AM, Sonntag KC, Isacson O, & Kosik KS (2006) Stem Cells24, 857-864.
38. Sempere LF, Freemantle S, Pitha-Rowe I, Moss E, Dmitrovsky E, & Ambros V (2004) Genome Biol5, R13.
39. Leucht C, Stigloher C, Wizenmann A, Klafke R, Folchert A, & Bally-Cuif L (2008) Nat Neurosci11, 641-648.
40. Shibata M, Kurokawa D, Nakao H, Ohmura T, & Aizawa S (2008) J Neurosci28, 10415-10421.
41. Packer AN, Xing Y, Harper SQ, Jones L, & Davidson BL (2008) J Neurosci28, 14341-14346.
42. Laneve P, Di Marcotullio L, Gioia U, Fiori ME, Ferretti E, Gulino A, Bozzoni I, & Caffarelli E (2007) Proc Natl Acad Sci U S A104, 7957-7962.
43. Plaisance V, Abderrahmani A, Perret-Menoud V, Jacquemin P, Lemaigre F, & Regazzi R (2006) J Biol Chem281, 26932-26942.
44. Mascaux C, Laes JF, Anthoine G, Haller A, Ninane V, Burny A, & Sculier JP (2009) Eur Respir J33, 352-359.
45. Roccaro AM, Sacco A, Chen C, Runnels J, Leleu X, Azab F, Azab AK, Jia X, Ngo HT, Melhem MR, et al. (2008) Blood*.*
46. Roman-Gomez J, Agirre X, Jimenez-Velasco A, Arqueros V, Vilas-Zornoza A, Rodriguez-Otero P, Martin-Subero I, Garate L, Cordeu L, San Jose-Eneriz E, et al. (2009) J Clin Oncol27, 1316-1322.

## Claims

1. Compound capable of interacting with the binding between microRNA-9 and/or microRNA-9* and their targets for the treatment of AML.

2. Compound capable of decreasing the cellular level of mature microRNA-9and/or microRNA-9* for the treatment of AML.

3. Method for the treatment of AML wherein a therapeutic composition is administered to a patient in need thereof comprising as an active ingredient a compound capable of interacting with the binding between microRNA-9and/or microRNA-9*and their targets.

4. Method for the treatment of AML wherein a therapeutic composition is administered to a patient in need thereof comprising as an active ingredient a compound capable of decreasing the cellular level of microRNA-9 and/or microRNA-9*.


SEQUENCE LISTING

<110>  Erasmus University Medical Center Rotterdam

<120>  METHOD FOR THE TREATMENT OF ACUTE MYELOID LEUKEMIA

<130>  066EPP0

<160>  2

<170>  PatentIn version 3.5

<210>  1
<211>  26
<212>  DNA
<213>  Artificial Sequence

<220>
<223>  primer

<400>  1
ggatgtctcg agtgttttct acaaag                                          26


<210>  2
<211>  25
<212>  DNA
<213>  Artificial Sequence

<220>
<223>  primer

<400>  2
gaagcagcgg ccgttgaggc tcctc                                           25

SEQUENCE LISTING

<110>  Erasmus University Medical Center Rotterdam

<120>  METHOD FOR THE TREATMENT OF ACUTE MYELOID LEUKEMIA

<130>  066EPP0

<160>  2

<170>  PatentIn version 3.5

<210>  1
<211>  26
<212>  DNA
<213>  Artificial Sequence

<220>
<223>  primer

<400>  1
ggatgtctcg agtgttttct acaaag                                          26


<210>  2
<211>  25
<212>  DNA
<213>  Artificial Sequence

<220>
<223>  primer

<400>  2
gaagcagcgg ccgttgaggc tcctc                                           25